# EUROPEAN PATENT APPLICATION

(11) **EP 4 648 056 A1**
(43) Date of publication of application: **12.11.2025**
(21) Application number: 25172996.8
(22) Date of filing: 28.04.2025
(51) Int. Cl.: G16H 10/40, G01N 35/00, G16H 40/63, G01N 35/04

(54) **SAMPLE PROCESSING MODULE AND METHOD FOR PROCESSING INFORMATION OF SAMPLE PROCESSING MODULE**

(30) Priority: 11.05.2024 CN 202410585272
(71) Applicant: Shenzhen New Industries Biomedical Engineering Co., Ltd., Shenzhen, Guangdong 518122 (CN)
(72) Inventor: HUANG, Tao, Shenzhen (CN); TANG, Junhui, Shenzhen (CN); CHEN, Hua, Shenzhen (CN); PAN, Jun, Shenzhen (CN); XIAO, Liyang, Shenzhen (CN); YANG, Zhixiong, Shenzhen (CN); PAN, Xiong, Shenzhen (CN); HUANG, Zhuoran, Shenzhen (CN)
(74) Representative: Office Freylinger

(57) **Abstract**

The present disclosure relates to a sample processing module and a method for processing information of sample processing module. The sample processing module includes: a sample storage device, the sample storage device including a plurality of drawers for storing sample tubes; a state detection device of each drawer, configured to detect a state of the drawer corresponding to the state detection device; a human-computer interaction device, wherein a display interface of the human-computer interaction device at least includes a first area, configured to display information of the drawer; and a processor, configured to acquire the state of the drawer, and switch from the currently displayed information of the drawer to highlighting the information of a first drawer in the first area in response to detecting that the first drawer is opened, wherein the first drawer is any drawer of the sample storage device.

## Description

### Technical Field

The present disclosure relates to the technical field of medical devices, and in particular, to a sample processing module and a method for processing information of the sample processing module.

### Background

In an assembly line, a sample input and output module is an important part of the entire system, which is responsible for the automatic, efficient and accurate loading and recovery of samples.

The sample input and output module is a functional module with the most user operations and interventions in the entire assembly line. For example, a user needs to remove the output samples from the sample input and output module, or place the samples to be input into the sample input and output module, etc. Therefore, how to improve the convenience for user operation in the process of operating the sample input and output module by the user is an urgent problem to be solved.

### Summary

Based on this, it is necessary to provide a sample processing module and a method for processing information of the sample processing module for the above technical problems, which can improve the convenience for operation.

A sample processing module includes: a sample storage device, a state detection device of each drawer, a human-computer interaction device, and a processor.

The sample storage device includes a plurality of drawers for storing sample tubes.

The state detection device of each drawer is configured to detect a state of the drawer corresponding to the state detection device.

A display interface of the human-computer interaction device at least includes a first area, configured to display information of the drawer.

The processor is configured to acquire the state of the drawer, and switch from currently displayed information of the drawer to highlighting a information of a first drawer in the first area in response to detecting that the first drawer is opened. The first drawer is any drawer of the sample storage device.

A method for processing information of a sample processing module includes the following operations.

A state of each drawer of the sample processing module is detected.

Currently displayed information of the drawer is switched to highlighting the information of the first drawer in a first area of a human-computer interaction device in response to detecting that the first drawer is opened.

The above sample processing module includes the sample storage device, the state detection device of each drawer, the human-computer interaction device, and the processor. The state detection device of each drawer is configured to detect the state of the drawer corresponding to the state detection device. The first area of the display interface of the human-computer interaction device is configured to display the information of the drawer. The processor acquires the state of the drawer, and switches from the currently displayed information of the drawer to highlighting the information of the first drawer in the first area in response to detecting that the first drawer is opened. Therefore, the action of opening the drawer can be associated with the switching action of a content displayed in the first area. In a case where a user performs an opening operation on the drawer of the sample processing module, the information of the opened drawer is highlighted, which can play a role in enlarging the information for prompting the opened drawer, thereby providing information prompts for user operation, and improving the convenience for user operation and the user experience.

### Brief Description of the Drawings

Fig. 1 is a schematic structural diagram of a sample processing module in some embodiments.
Fig. 2 is a schematic diagram of displaying information of each drawer of a sample storage device in a balanced manner in some embodiments.
Fig. 3 is a schematic diagram of switching a content displayed in a first area in some embodiments.
Fig. 4 is a schematic diagram of switching a content displayed in a first area in other embodiments.
Fig. 5 is a schematic diagram of switching a content displayed in a first area in still other embodiments.
Fig. 6 is a schematic diagram of displaying information of each drawer of a sample storage device in a balanced manner in still other embodiments.
Fig. 7 is a schematic diagram of highlighting information of a normal sample output drawer in some embodiments.
Fig. 8 is a schematic diagram of a first information page of an error sample output drawer in some embodiments.
Fig. 9 is a schematic diagram of a display interface of a human-computer interaction device in some embodiments.
Fig. 10 is a schematic flowchart of a method for processing information of a sample processing module in some embodiments.
Fig. 11 is an internal structural diagram of a computer device in some embodiments.

### Detailed Description of the Embodiments

In order to make the purposes, technical solutions and advantages of the present disclosure clearer, the present disclosure will be further described below in detail in conjunction with the drawings and embodiments. It is to be understood that that the specific embodiments described herein are only used to illustrate the present disclosure, but are not intended to limit the present disclosure.

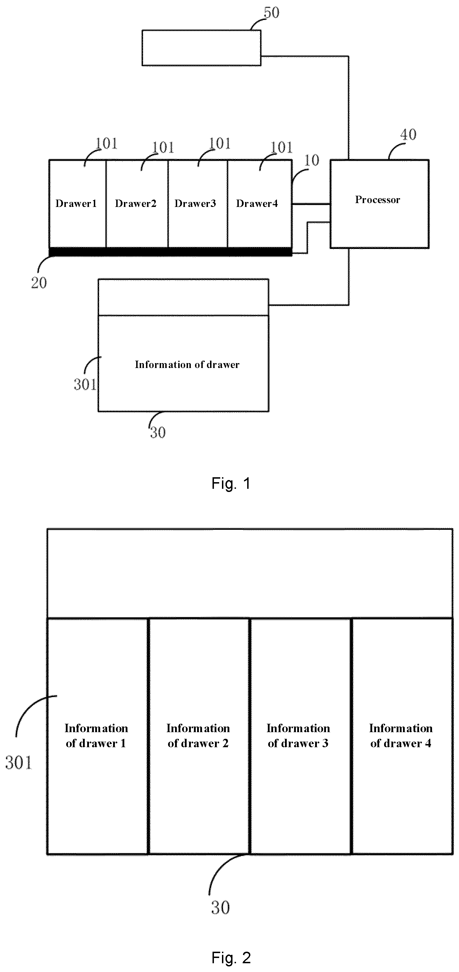

A sample processing module provided in the present disclosure, as shown in Fig. 1, includes: a sample storage device 10, a state detection device 20 of each drawer, a human-computer interaction device 30, and a processor 40.

The sample storage device 10 includes a plurality of drawers 101 for storing sample tubes.

The state detection device 20 of each drawer is configured to detect a state of the drawer corresponding to the state detection device.

A display interface of the human-computer interaction device at least includes a first area 301, configured to display information of the drawer.

The processor 40 is configured to switch from the currently displayed information of the drawer to highlighting the information of a first drawer in the first area in response to detecting that the first drawer is opened. The display interface of the human-computer interaction device 30 may be configured to display the information of the sample processing module, and the first area 301 is an information display area corresponding to the sample storage device 10, which is configured to display the information of the drawer. By displaying the information of the drawer in the first area 301 of the human-computer interaction device 30, a user can intuitively know the information of the drawer of the sample storage device 10. The information of the drawer includes, but is not limited to, the state of the drawer and a storage state of a sample in the drawer. The first area 301 may be configured to display the information of all the drawers, or the information of some drawers (for example, one).

In some embodiments, the state detection device 20 may include a sensing piece and a triggering piece. The triggering piece is arranged below a mounting plate of the drawer, and the sensing piece is arranged in a closed position of the drawer. In response to the drawer being pushed in, the sensing piece can sense the triggering piece below the mounting plate of the drawer, thereby determining that the drawer is in a closed state. In response to the sensing piece not sensing the triggering piece below the mounting plate, it indicates that the drawer is in an open state.

The processor 40 is in communication connection with the state detection device 20 of each drawer to acquire the state of each drawer.

In some embodiments, a locking piece (not shown in the figure) may be further arranged below the mounting plate of the drawer, and the locking piece is in communication connection with the processor 40. The processor controls the locking piece to lock the drawer in response to detecting that the drawer is in the closed position, thereby preventing the drawer from being accidentally opened in the operation process. At this time, the drawer is in a locked state. In response to the locking piece being unlocked and the drawer being pulled out, the sensing piece no longer senses the triggering piece below the mounting plate, which indicates that the drawer is in an unlocked state. In some embodiments, the locked state of the drawer represents that the drawer is closed and in the closed state. The unlocked state of the drawer represents that the drawer is opened and in the open state.

In some embodiments, the information of the drawer displayed in the first area is related to the current state of the drawer in the sample storage device, and is configured to display the relevant state of the drawer to the user in real time. For example, in a case where all the drawers are in the closed state, the information of each drawer of the sample storage device is displayed in the first area. In a case where one of the drawers is opened, the information of the opened drawer is displayed in the first area.

In a case where the user pulls the drawer out from the closed position, the drawer changes from the closed state to the open state, that is, the drawer is opened. The processor switches from the currently displayed information of the drawer to highlighting the information of the first drawer in the first area in response to detecting that the first drawer is opened.

The first drawer is any drawer of the sample storage device, and is configured to represent the currently operated drawer in the sample storage device. For example, in a case where the first drawer in the sequence of the sample storage device is opened or closed, the drawer may be represented as the first drawer, and the currently displayed information of the drawer is switched to highlighting the information of the first drawer in the first area.

The information of the opened drawer is highlighted, so that the information of the opened drawer becomes obvious. In a highlighting manner, the currently displayed information of the drawer may be hidden and only the information of the opened first drawer is displayed in the first area. In a highlighting manner, a second layer may be created over a first layer of the currently displayed information of the drawer, the second layer is configured to display the information of the opened first drawer, the second layer is placed in the middle of the first area, and the transparency of the first layer is increased, thereby playing the role of highlighting the information of the first drawer on the second layer.

The processor switches from the currently displayed information to highlighting the information of the first drawer through in the first area in response to detecting that the first drawer is opened, so that the action of opening the drawer can be associated with the switching action of a content displayed in the first area. For the user, the information of the opened first drawer is highlighted, which can play a role in enlarging the information for prompting the opened drawer, so that the user can focus on the currently opened drawer, thereby improving the convenience for user operation and the user experience.

In some embodiments, the sample storage device may include an error sample output drawer for storing sample tubes with detection abnormalities in an assembly line inspection process. For the user, in the inspection process, there is a need to manually check the sample tubes in the error sample output drawer, so that it is necessary to frequently pull out the error sample output drawer to check the sample tubes. In a case where the user pulls out the error sample output drawer, the user can obtain an information prompt for the error sample output drawer by highlighting the information of the opened error sample output drawer in the first area of the human-computer interaction device. In some embodiments, the information of the error sample output drawer includes placement positions of different types of sample tubes with detection abnormalities in the error sample output drawer. The user can know from the information of the error sample output drawer where to find a target sample tube in the error sample output drawer, thereby improving the convenience for operation.

The above sample processing module includes the sample storage device, the state detection device of each drawer, the human-computer interaction device, and the processor. The state detection device of each drawer is configured to detect the state of the drawer corresponding to the state detection device. The first area of the display interface of the human-computer interaction device is configured to display the information of the drawer. The processor switches from the currently displayed information of the drawer to highlighting the information of the first drawer in the first area in response to detecting that the first drawer is opened. Therefore, the action of opening the drawer can be associated with the switching action of the content displayed in the first area. In a case where the user performs an opening operation on the drawer of the sample processing module, the information of the opened drawer is highlighted, which can play a role in enlarging the information for prompting the opened drawer, thereby providing the information prompts for user operation, and improving the convenience for user operation and the user experience.

In some embodiments, in a case where all the drawers of the sample storage device are in the closed state, the first area is configured to display the information of each drawer of the sample storage device in a balanced manner.

The information of each drawer of the sample storage device is displayed in a balanced manner, which means that the information of each drawer of the sample storage device can be displayed in equal proportion, so that the information of each drawer can be prompted without distinction from the display effect.

In some embodiments, the first area is divided into a corresponding number of sub-areas in equal proportion according to the number of drawers, each sub-area corresponds to the drawer according to the layout of the actual physical position of the drawer, and the sub-area is configured to display the drawer information of the drawer corresponding to the state detection device. That is, the layout of the sub-area in the first area is consistent with the layout of the drawer in the sample storage device. As shown in Fig. 2, according to the four drawers in the sample storage device, the first area is divided into four sub-areas, and each sub-area corresponds to the drawer according to the layout of the actual physical position of each drawer. In order of the interface from left to right, the first sub-area is configured to display the information of drawer 1, the second sub-area is configured to display the information of drawer 2, the third sub-area is configured to display the information of drawer 3, and the fourth sub-area is configured to display the information of drawer 4.

It should be understood that, in order to display the information of each drawer of the sample storage device in a balanced manner, the layout of each sub-area in the first area is not limited to the example shown in Fig. 2. The division and layout of each sub-area of the first area can be consistent with the number and layout of the drawers of the sample storage device.

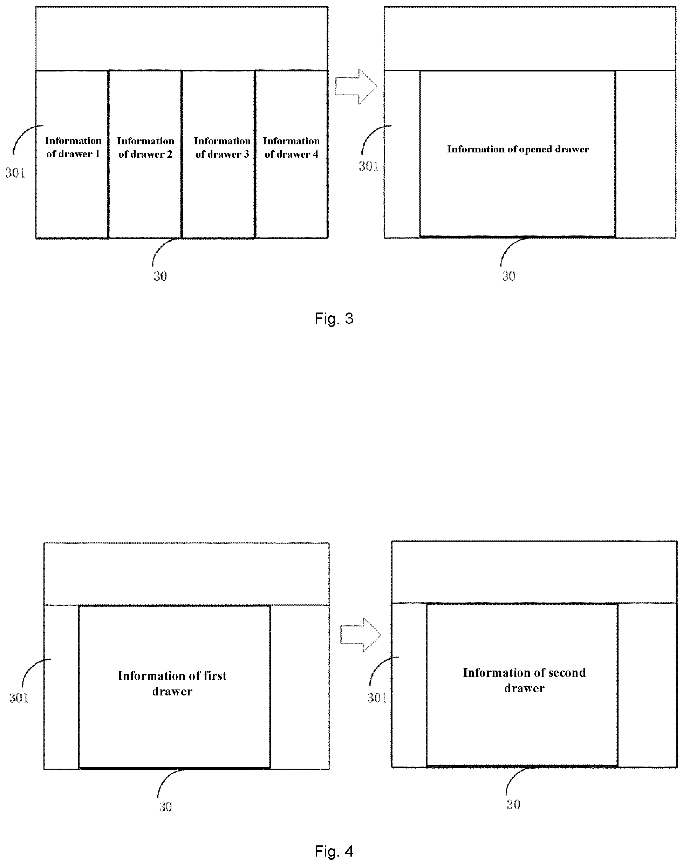

As shown in Fig. 3, the processor is further configured to switch, in a case where all the drawers of the sample storage device are closed, from displaying the information of each drawer of the sample storage device in a balanced manner to highlighting the information of the first drawer in the first area in response to detecting that the first drawer is opened.

In some embodiments, in a case where all the drawers of the sample storage device are in the closed state, the information of each drawer of the sample storage device is displayed in a balanced manner in the first area, and the information of each drawer can be prompted without distinction. The processor switches from displaying the information of each drawer of the sample storage device in a balanced manner to highlighting the information of the opened first drawer in response to detecting that the first drawer is opened, which can play a role in enlarging the information for prompting the opened first drawer, so that the user can focus on the currently opened drawer.

In some embodiments, the processor is further configured to switch, in a case where a second drawer is already open in the sample storage device, from highlighting the drawer information of the second drawer to highlighting the information of the first drawer in the first area in response to detecting that the first drawer is opened.

In some embodiments, the action of opening the drawer is associated with the switching action of the content displayed in the first area. In response to the first drawer being opened, the information of the first drawer is highlighted in the first area of the human-computer interaction device, which can play a role in enlarging the information for prompting the opened drawer, so that the user can focus on the currently opened drawer. In a case where the second drawer is already open, in response to the first drawer being opened, highlighting the drawer information of the last opened second drawer is switched to highlighting the information of the most recently opened first drawer in the first area, so that the information of the most recently opened first drawer is enlarged, and the user can focus on the most recently opened drawer.

As shown in Fig. 4, in a case where drawer 1 is opened, the information of drawer 1 is highlighted in the first area. In a case where drawer 1 is already open, in response to drawer 2 being opened, highlighting the information of drawer 1 is switched to highlighting the information of drawer 2 in the first area. In this way, each time the new drawer is opened, the information of the most recently opened drawer is enlarged, so that the user can focus on the most recently opened drawer.

In some embodiments, the processor is further configured to switch from highlighting the information of the first drawer to displaying the information of each drawer of the sample storage device in a balanced manner in the first area in response to detecting that the first drawer is closed and all the drawers of the sample storage device are in the closed state after the first drawer is closed.

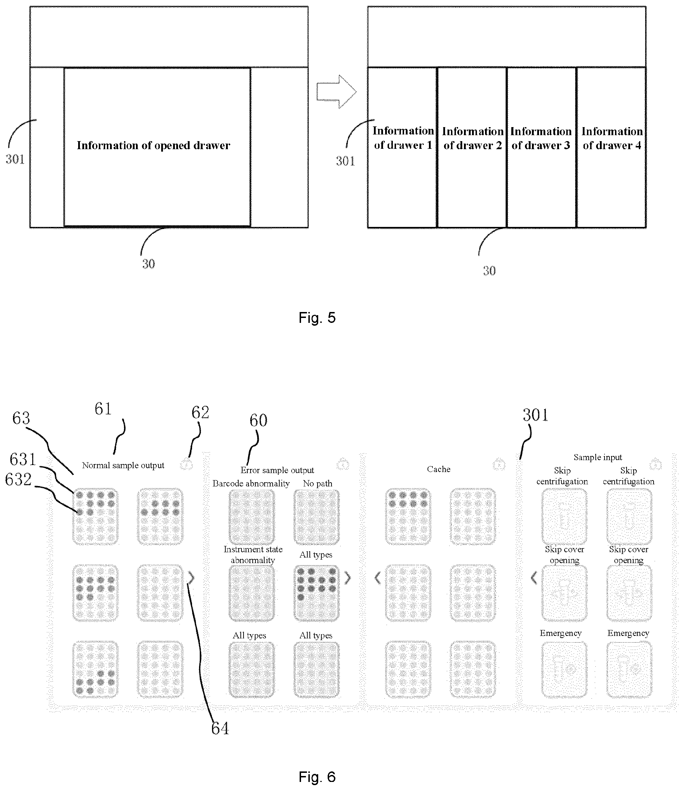

As shown in Fig. 5, in a case where drawer 1 is opened, the information of drawer 1 is highlighted in the first area. In response to drawer 1 being pushed to the closed position and all the drawers of the sample storage device being in the closed state, highlighting the information of drawer 1 is switched to displaying the information of each drawer of the sample storage device in a balanced manner in the first area. In this way, the action of opening the drawer is associated with the switching action of the content displayed in the first area. In response to the drawer being opened, the information of the drawer is highlighted in the first area of the human-computer interaction device, which can play a role in enlarging the information for prompting the opened drawer, so that the user can focus on the currently opened drawer. In response to the drawer being pushed to the closed position and all the drawers being in the closed state, the information of each drawer of the sample storage device is displayed in a balanced manner, so that the information of each drawer can be prompted without distinction.

In some embodiments, the processor is configured to switch, in a case where the second drawer is already open in the sample storage device, from highlighting the information of the second drawer to highlighting the information of the first drawer in the first area in response to detecting that the first drawer is opened, and switch, in a case where the number of the second drawers that are already open is one, from highlighting the information of the first drawer to highlighting the information of the second drawer in the first area in response to detecting that the first drawer is closed.

In some embodiments, the second drawer is already in the open state before the first drawer is opened, that is, the opening time of the second drawer is before that of the first drawer, and when the first drawer is opened, the second drawer is still in the open state. As shown in Table 1, when the user opens the second drawer at a first time, the information of the second drawer is highlighted in the first area. When the user opens the first drawer at a second time, highlighting the information of the second drawer is switched to highlighting the information of the first drawer in the first area. As shown in Fig. 4, in a case where the first drawer is closed before the second drawer, that is, when the user closes the first drawer at a third time, highlighting the information of the first drawer is switched to highlighting the information of the second drawer in the first area.

**Operation Timing Table 1**

| Time | First time | Second time | Third time |
|---|---|---|---|
| Action | Open the second drawer | Open the first drawer | Close the first drawer |
| Content displayed in first area | Highlight information of the second drawer | Highlight information of the first drawer | Highlight information of the second drawer |

In this way, the action of opening the drawer is associated with the switching action of the content displayed in the first area. In response to the drawer being opened, the information of the drawer is highlighted in the first area of the human-computer interaction device, which can play the role of enlarging the information for prompting the opened drawer, so that the user can focus on the currently opened drawer. In response to the drawer being pushed to the closed position and another drawer being still in the open state, the information of the drawer that is still in the open state is highlighted, and the information for prompting the opened drawer is continued to be enlarged.

In some embodiments, the processor is configured to switch, in a case where the second drawer is already open in the sample storage device, from highlighting the information of the second drawer to highlighting the information of the first drawer in the first area in response to detecting that the first drawer is opened, and switch, in a case where the number of the second drawers that are already open is at least two, from highlighting the information of the first drawer to highlighting the information of a target drawer in the first area in response to detecting that the first drawer is closed. The target drawer is either the drawer whose opening time is closest to the current time among the at least two second drawers or the drawer with the highest priority among the at least two second drawers.

In some embodiments, the second drawer is already in the open state before the first drawer is opened, that is, the opening time of the at least two second drawers is before that of the first drawer, and when the first drawer is opened, the at least two second drawers are still in the open state. As shown in Table 2, when the user opens the second drawer (1) at a first time, the information of the second drawer (1) is highlighted in the first area. When the user opens the second drawer (2) at a second time, the information of the second drawer (2) is highlighted in the first area. When the user opens the first drawer at a third time, highlighting the information of the second drawer (2) is switched to highlighting the information of the first drawer in the first area. In a case where the user closes the first drawer at a fourth time, the content displayed in the first area should change because the state of the first drawer changes. At this time, the processor may determine the target drawer among the at least two second drawers that are still in the open state, and switch from highlighting the information of the first drawer to highlighting the information of the target drawer in the first area. The target drawer is either the drawer whose opening time is closest to the current time among the at least two second drawers or the drawer with the highest priority among the at least two second drawers.

**Operation Timing Table 2**

| Time | First time | Second time | Third time | Fourth time |
|---|---|---|---|---|
| Action | Open the second drawer (1) | Open the second drawer (2) | Open the first drawer | Close the first drawer |
| Content displayed in first area | Highlight information of the second drawer (1) | Highlight information of the second drawer (2) | Highlight information of the first drawer | Highlight information of the target drawer |

For example, the opening time of the second drawer (2) is closest to the current time among the at least two second drawers opened first, so that highlighting the information of the first drawer may be switched to highlighting the information of the second drawer (2) in the first area. In this way, in a case where the opened drawer is pushed in, by switching to highlighting the information of the last opened drawer in the first area, the information of the last opened drawer is highlighted, and the information for prompting the opened drawer is continued to be enlarged.

For another example, the priority of the second drawer (1) is the highest among the at least two second drawers opened first, so that highlighting the information of the first drawer may be switched to highlighting the information of the second drawer (1) in the first area. The priority of the drawer may be classified according to the type of the drawer, or user-defined according to the degree of attention. For example, the priority of the error sample output may be set to be the highest. In a case where the at least two drawers opened first include the second drawer (1) (the error sample output drawer), highlighting the information of the first drawer may be switched to highlighting the information of the second drawer (1) (the error sample output drawer) in the first area. In this way, in a case where the opened drawer is pushed in, by switching to highlighting the information of the more concerned drawer in the first area, the information of the concerned drawer in the drawer that is still in the open state is highlighted, and the information for prompting the concerned drawer is continued to be enlarged.

In some embodiments, the processor is configured to switch, in a case where the second drawer is already open in the sample storage device, from highlighting the information of the second drawer to highlighting the information of the first drawer in the first area in response to detecting that the first drawer is opened, and remain the content displayed in the first area unchanged in response to detecting that the second drawer is closed.

In some embodiments, the second drawer is already in the open state before the first drawer is opened, that is, the opening time of the second drawer is before that of the first drawer, and when the first drawer is opened, the second drawer is still in the open state. As shown in Table 3, when the user opens the second drawer at a first time, the information of the second drawer is highlighted in the first area. When the user opens the first drawer at a second time, highlighting the information of the second drawer is switched to highlighting the information of the first drawer in the first area. in a case where any second drawer is closed before the first drawer, that is, the user closes the second drawer at a third time, the content displayed in the first area remains unchanged, and the information of the first drawer is still highlighted.

**Operation Timing Table 3**

| Time | First time | Second time | Third time |
|---|---|---|---|
| Action | Open the second drawer | Open the first drawer | Close the second drawer |
| Content displayed in first area | Highlight information of the second drawer | Highlight information of the first drawer | Highlight information of the first drawer |

In some embodiments, the drawer is configured to accommodate a sample carrier, and the sample carrier includes a plurality of placement positions for loading the sample tubes. In some embodiments, the sample carrier may be a sample box or a sample rack. The sample carrier is provided with the plurality of placement positions for loading the sample tubes, which may be sample holes.

In order to provide rich information of the drawer in the first area, In some embodiments, the information of the drawer includes at least one of a type of the drawer, the state of the drawer, a type of the sample carrier in the drawer, a state of the sample carrier in the drawer, a layout of the sample carrier in the drawer, a state of each placement position in the sample carrier, and a layout of the placement position in the sample carrier.

The type of the drawer is user-defined, and distinguished by setting a name for each drawer. For example, drawer 1 may be set as a normal sample output drawer, which is configured to store the sample tubes that are normally output on an assembly line. Drawer 2 may be set as an error sample output drawer, which is configured to store the sample tubes with detection abnormalities on the assembly line. Drawer 3 is set as a cache drawer, which is configured to cache the sample tubes. Drawer 4 is set as a sample input drawer, which is configured to store the sample tubes for sample input. In some embodiments, the type of the drawer may be defined on the human-computer interaction device, or may be defined on assembly line middleware and then synchronized to the human-computer interaction device for display.

The state of the drawer may include a closed state and an open state. In some embodiments, the closed state of the drawer corresponds to the locked state of the drawer, and the open state of the drawer corresponds to the unlocked state of the drawer.

The state of the sample carrier in the drawer represents whether the sample carrier is placed in a carrier position in the drawer. The state of the sample carrier in the drawer includes a placement state of the sample carrier in each carrier position. In some embodiments, the sample processing module is provided with a detection sensor in each carrier position of the drawer, which is configured to detect whether the sample carrier is placed in the carrier position. In a case where the user places the sample carrier in the carrier position, the detection sensor senses a signal to the processor, and the processor records the carrier position as having the sample carrier according to the signal. For example, six carrier positions are arranged in the drawer, which may be configured to place six sample carriers. In a case where the detection sensors corresponding to five of the carrier positions detect the sample carriers, and the detection sensor corresponding to one of the carrier positions does not detect the sample carrier, it indicates that the sample carriers are placed in five of the carrier positions, and the sample carrier is not placed in one of the carrier positions. In this way, the information of the drawer, which is displayed in the first area, includes the state of the sample carrier, and the user may directly know which carrier positions in the drawer the sample carriers are placed in, so as to be convenient for user operation.

The type of the sample carrier in the drawer is configured to classify the sample carriers in the drawer, so that the sample tube of the specified type is placed in the corresponding sample carrier for storage. For example, the type of the sample carrier in the error sample output drawer may be set to include: barcode abnormality, no path, instrument state abnormality, etc. The sample tube with barcode abnormality is stored in the sample carrier corresponding to the barcode abnormality, and the sample tube with no path is stored in the sample carrier corresponding to the no path. The type of the sample carrier may be defined on the human-computer interaction device, or may be defined on the assembly line middleware and then synchronized to the human-computer interaction device for display.

The layout of the sample carrier in the drawer is configured to represent the carrier position of the sample carrier placed in the drawer. For example, in a case where the sample carriers are placed in five carrier positions, the specific five carrier positions the sample carriers are placed in may be known through the layout of the sample carriers.

The state of each placement position in the sample carrier represents whether the sample tube is placed in the placement position.

The layout of each placement position in the sample carrier represents the position of the placement position of the sample carrier, the number of the placement positions, the shape and size of the sample carrier, etc.

In some embodiments, the above information of the drawer may be expressed in text form and displayed in the first area.

In some embodiments, in order to more vividly and intuitively represent the above information of the drawer, the first area may be configured to display a drawer information page, and the information of the drawer is recorded in the drawer information page. The drawer information page includes a drawer name, a drawer state icon, and a layout diagram of the drawer.

The drawer name is configured to represent the type of the drawer.

The drawer state icon is configured to represent the state of the drawer.

The layout diagram of the drawer includes the type of the sample carrier, a sample carrier icon, and a placement position icon. A display element of the sample carrier icon is configured to represent the state of the actual sample carrier. A display element of the placement position icon is configured to represent the state of the actual placement position. The layout of the sample carrier icon corresponds to the layout of the actual sample carrier in the drawer. The layout of the placement position corresponds to the layout of the actual placement position in the sample carrier. In some embodiments, as shown in Fig. 6, the first area 301 is configured to display the drawer information page, and the information of the drawer is recorded in the drawer information page. The information page 60 of one drawer is shown in Fig. 6, including a drawer name 61, a drawer state icon 62, and a layout diagram 63 of the drawer.

The layout diagram of the drawer includes a sample carrier icon 631 and a placement position icon 632. A display element of the sample carrier icon is configured to represent the state of the actual sample carrier. A display element of the placement position icon is configured to represent the state of the actual placement position. A layout of the sample carrier icon corresponds to the layout of the actual sample carrier in the drawer. A layout of the placement position corresponds to the layout of the actual placement position in the sample carrier.

The sample carrier icon 631 may represent the state of the actual sample carrier by its color. For example, the outline of the sample carrier icon 631 in a normal operating state is displayed in gray, and the outline of the sample carrier icon 631 in an abnormal operating state is displayed in red, so that the operating state of the sample carrier is distinguished by the color of the outline of the sample carrier icon 631.

The states of the placement positions in the sample carrier may be distinguished by different colors. For example, gray represents that no sample tube is loaded, red represents abnormal loading, and green represents that the sample tube is loaded.

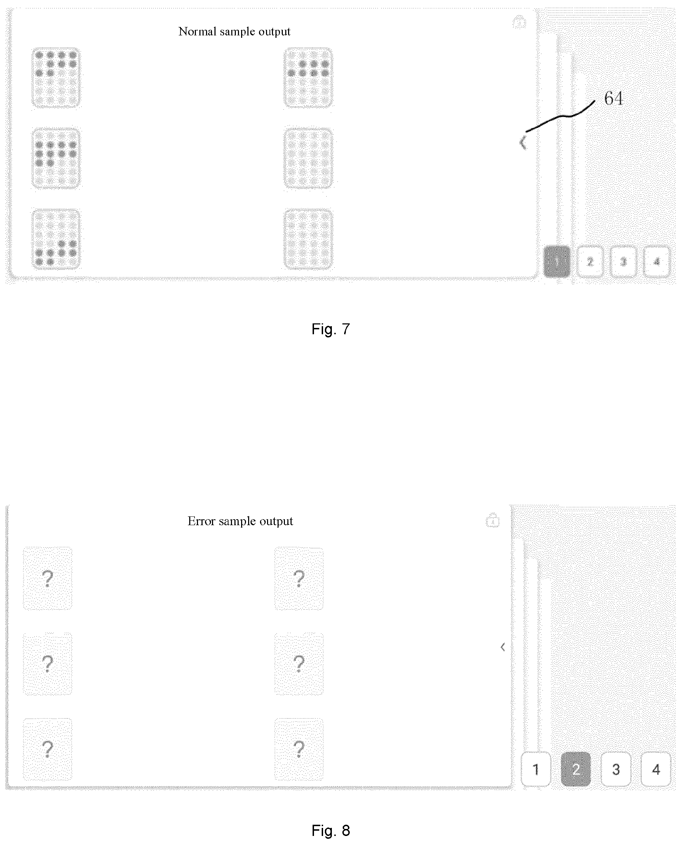

In some embodiments, as shown in Fig. 6, the information page of the drawer further includes a display mode switching control 64. Displaying the information page of each drawer in a balanced manner is switched to highlighting the information page of the corresponding drawer in the first area in response to detecting an operation on the display mode switching control of the information page of any drawer in a balanced display state. For example, on the basis of displaying the drawer information page of each drawer in a balanced manner shown in Fig. 6, after a triggering operation on the display mode switching control of the drawer information page of the normal sample output drawer shown in Fig. 6 is detected, displaying in a balanced manner shown in Fig. 6 is switched to highlighting the drawer information page of the normal sample output drawer shown in Fig. 7 in the first area.

In some embodiments, as shown in Fig. 7, the information page of the drawer further includes a display mode switching control 64. Highlighting the information page of the current drawer is switched to displaying the information page of each drawer in the first area in a balanced manner in response to detecting an operation on the display mode switching control of the information page of any drawer in a highlighted state. For example, on the basis of highlighting the drawer information page of the sample output drawer shown in Fig. 7, in response to the operation on the display mode switching control of the information page being detected, highlighting shown in Fig. 7 is switched to displaying the drawer information page of each drawer in a balanced manner shown in Fig. 6 in the first area.

In some embodiments, various display requirements of the user may be met through the display mode switching control.

In some embodiments, the drawer is configured to accommodate a sample carrier, and the sample carrier includes a plurality of placement positions for loading the sample tubes. The first area is configured to display a drawer information page, and the information of the drawer is recorded in the drawer information page.

The drawer information page includes a first information page and a second information page. The first information page and the second information page have different application scenarios and interfaces.

The processor is further configured to highlight the first information page of the opened first drawer in response to the first drawer being opened. In actual operation, in a case where the drawer is opened, the user can operate the sample carrier and the sample tube in the drawer, for example, take the entire sample carrier or take the sample tube from the sample carrier. At this time, if the information before the drawer is opened is displayed in the first information page, the displayed drawer information is inaccurate, which may cause inconvenience to the user.

Based on this, in some embodiments, the highlighted first information page is shown in Fig. 8, and the first information page includes at least one of a type of the drawer, a state icon of the drawer, a type of the sample carrier in the drawer, and an unknown state icon of the sample carrier in the drawer. The unknown state icon of the sample carrier represents an unknown state of the sample carrier in the drawer.

In a case where all the drawers are in the closed state, the processor is configured to display the second information page of each drawer of the sample storage device in a balanced manner, and the second information page includes a drawer name, a drawer state icon, and a layout diagram of the drawer. The drawer state icon is configured to represent the state of the drawer.

The second information page in some embodiments is shown in Fig. 6, and the second information page includes a drawer name, a drawer state icon, and a layout diagram of the drawer. The drawer name is configured to represent the type of the drawer. The drawer state icon is configured to represent the state of the drawer.

The layout diagram of the drawer includes the type of the sample carrier, a sample carrier icon, and a placement position icon. A display element of the sample carrier icon is configured to represent the state of the actual sample carrier. A display element of the placement position icon is configured to represent the state of the actual placement position. The layout of the sample carrier icon corresponds to the layout of the actual sample carrier in the drawer. The layout of the placement position corresponds to the layout of the actual placement position in the sample carrier.

In some embodiments, the first information page that is highlighted in a case where the drawer is opened and the second information page that is displayed in a balanced manner in a case where all the drawers are in the closed state have different information contents. The first information page displays the unknown state icon of the sample carrier in the drawer, and the second information page displays the layout diagram of the drawer, which can provide correct information prompts for the user in different states and avoid interference from erroneous information.

In some embodiments, a manner of highlighting the information of the drawer may be as follows: the information to be highlighted of the drawer is enlarged and displayed, and the information of the other drawers is partially or completely covered, or the information to be highlighted of the drawer is enlarged and displayed, and the information of the other drawers is stacked below the enlarged and displayed information of the drawer.

For example, the information page to be highlighted of the drawer is enlarged and displayed, and the information pages of other drawers are partially or completely covered, or the information page to be highlighted of the drawer is enlarged and displayed, and the information pages of other drawers are stacked below the enlarged and displayed information page of the drawer.

It is understandable that the implementation of the highlighted information of the drawer here may be configured to highlight the information of the first drawer mentioned above, and may also be configured to highlight the information of the second drawer mentioned above. For the sample processing module, any of the above manners of highlighting the drawer information may be implemented using the implementations described in some embodiments.

In some embodiments, the first area further includes a drawer switching identifier. As shown in Fig. 8, the drawer switching identifier may be represented by a digital number corresponding to the drawer. In response to the user triggering a certain digital number, the drawer information page of the drawer corresponding to the digital number is switched in the first area.

In some embodiments, the operation of displaying the information of each drawer of the sample processing module in a balanced manner includes: dividing the first area into a corresponding number of sub-areas in equal proportion according to the number of drawers of the sample storage device; and according to the physical position of the drawer, enabling each sub-area to correspond to the drawer, and displaying the information page of the corresponding drawer in each sub-area.

As shown in Fig. 6, according to the four drawers in the sample storage device, the first area is divided into four sub-areas in equal proportion, and according to the layout of the actual physical position of each drawer, each sub-area corresponds to the drawer. In order of the interface from left to right, the first sub-area is configured to display the information of the normal sample output drawer, the second sub-area is configured to display the information of the error sample output drawer, the third sub-area is configured to display the information of the cache drawer, and the fourth sub-area is configured to display the information of the sample input drawer.

In this way, while achieving balanced display of the information of each drawer, the information can also correspond to the physical position of the drawer, which is convenient for the user to intuitively obtain the drawer information.

It is understandable that, compared with the balanced display of the information of each drawer of the sample storage device, when the information of the opened drawer is highlighted, there is more space for the information of the drawer to be displayed, so that compared with the balanced display of the information of the plurality of drawers, the information of the opened drawer can be expanded by highlighting the information of the opened drawer. From the display effect, in addition to being able to play a role in highlighting the information, it can also play a role in displaying the information, so as to display richer information. For example, when the information of the plurality of drawers is displayed in a balanced manner, due to the limited display space allocated, the name of the sample carrier cannot be fully displayed when it is relatively long, and after the drawer is pulled out, the information of the drawer is highlighted, so that in this state, the name of the sample carrier in the drawer can be fully displayed, which is convenient for the user to check.

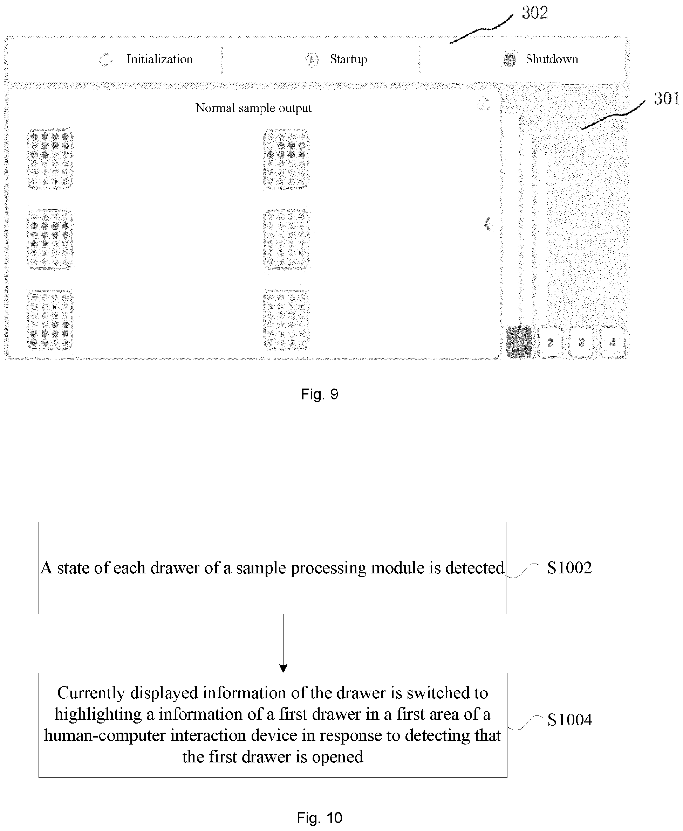

In some embodiments, as shown in Fig. 9, the display interface of the human-computer interaction device further includes a second area 302 for displaying an operation control. The operation control includes at least one of an initialization control, a startup control, and a shutdown control.

The initialization control is configured to control the sample processing module to perform an initialization operation. The startup control is configured to control the sample processing module to perform a startup operation, and the shutdown control is configured to control the sample processing module to perform a shutdown operation.

Through the operation control in the second area, the user can conveniently operate the sample processing module through the human-computer interaction device.

In some embodiments, as shown in Fig. 1, the sample processing module further includes a mechanical arm 50. The type of the drawer includes a sample output drawer.

The processor is further configured to control the mechanical arm to count the state of the placement position of the sample carrier of the sample output drawer in response to detecting that the opened sample output drawer is closed, and update the information of the sample output drawer according to a counting result.

The sample output drawer includes a normal sample output drawer and an error sample output drawer. Both the normal sample output drawer and the error sample output drawer are configured to achieve the sample output processing of the sample tube on the assembly line. Sample output means that the sample tube that has completed the test on the assembly line is withdrawn from the assembly line and stored in the sample output drawer.

In some embodiments, in response to the sample output drawer in the open state being pushed to the closed position, a counting operation is triggered. The reason for setting the counting is that the user often needs to operate the sample output drawer, for example, take out the sample carrier filled with sample tubes and replace the empty sample carrier. That is, the state of the sample carrier in the sample output drawer is changing.

In order to accurately record the drawer information in the sample output drawer, in response to the sample output drawer in the open state being pushed to the closed position, the counting operation is triggered, the mechanical arm is controlled to count the state of the placement position of the sample carrier of the sample output drawer, and the information of the sample output drawer is updated according to the counting result. In this way, in a case where the sample output drawer changes from the open state to the closed state, the information of the sample output drawer may be accurately recorded through the counting operation.

In some embodiments, the processor is configured to control the mechanical arm to count a target placement position of the sample carrier storing the sample tube in the sample output drawer, and detect whether the sample tube still exists in the target placement position. The target placement position is any placement position in the sample carrier where the sample tube is placed.

In some embodiments, according to the operating habits of the user, the user usually replaces the sample carrier in the drawer as a whole. Therefore, the object of the counting operation is the sample carrier where the sample tube is placed in the drawer, and the placement position where the sample tube is placed in the sample carrier. For example, the processor records that the sample tubes are placed in all the placement positions of drawer 2 in the drawer, and the sample tube is placed in the second placement position of drawer 3. No sample tubes are placed in the other placement positions in the drawer.

In response to the sample output drawer being pushed from the open state to the closed position, the processor controls the mechanical arm to count any placement position of drawer 2 and drawer 3 where the sample tube is placed, and detects whether the sample tube still exists in the placement position.

Generally speaking, during sample output, the mechanical arm places the sample tubes in a certain order. In order to improve the counting efficiency, the mechanical arm may count the first placement position where the sample tube is placed in the sample carrier according to the placement order. After counting the first placement position where the sample tube is placed in the sample carrier, the mechanical arm moves to the first placement position where the sample tube is placed in the next sample carrier for counting.

In some embodiments, the processor is configured to update the state of each placement position of the sample carrier to no sample tube in a case where no sample tube is detected in the target placement position of the sample carrier storing the sample tube in the sample output drawer.

That is, after counting the first placement position of the sample carrier where the sample tube is placed, in a case where there is no sample tube in the placement position, it may be determined that the user has replaced the sample carrier. At this time, the state of each placement position of the sample carrier in the carrier position is updated to no sample tube.

Through the counting operation, the information of the sample output drawer may be accurately updated. It is understandable that in a case where the information of the drawer is recorded through the drawer information page, if the state of the sample carrier changes after the counting, the color of the display icon of the placement position in the sample carrier is updated in the corresponding layout diagram to show the change of the counting result.

Based on the same inventive concept, as shown in Fig. 10, the present disclosure further provides a method for processing information of a sample processing module, which is applied to the processor shown in Fig. 1, and includes the following operations.

At S1002, a state of each drawer of the sample processing module is detected.

At S1004, currently displayed information of the drawer is switched to highlighting the information of a first drawer in a first area of a human-computer interaction device in response to detecting that the first drawer is opened.

In some embodiments, the sample processing module includes a sample storage device, the sample storage device includes a plurality of drawers for storing sample tubes, and the method for processing information of the sample processing module further includes: in a case where all the drawers of the sample storage device are in a closed state, switching from displaying the information of each drawer of the sample storage device in a balanced manner to highlighting the information of the first drawer in the first area in response to detecting that the first drawer is opened.

In some embodiments, the method for processing information of the sample processing module further includes: in a case where the second drawer is already open in the sample storage device, switching from highlighting the information of the second drawer to highlighting the information of the first drawer in the first area in response to detecting that the first drawer is opened.

In some embodiments, the method for processing information of the sample processing module further includes: in a case where the number of the second drawers that are already open is one, switching from highlighting the information of the first drawer to highlighting the information of the second drawer in the first area in response to detecting that first drawer is closed.

In some embodiments, the method for processing information of the sample processing module further includes: in a case where the number of the second drawers that are already open is at least two, switching from highlighting the information of the first drawer to highlighting the information of a target drawer in the first area in response to detecting that the first drawer is closed. The target drawer is either the drawer whose opening time is closest to the current time among the at least two second drawers or the drawer with the highest priority among the at least two second drawers.

In some embodiments, the method for processing information of the sample processing module further includes: switching from highlighting the information of the first drawer to displaying the information of each drawer of the sample storage device in a balanced manner in the first area in response to detecting that the opened first drawer is closed and all the drawers of the sample storage device are in the closed state after the first drawer is closed.

In some embodiments, the drawer is configured to accommodate a sample carrier, and the sample carrier includes a plurality of placement positions for loading the sample tubes. The information of the drawer includes at least one of a type of the drawer, the state of the drawer, a type of the sample carrier in the drawer, a state of the sample carrier in the drawer, a layout of the sample carrier in the drawer, and a state of each placement position in the sample carrier.

In some embodiments, the first area is configured to display an information page of the drawer, and the information of the drawer is recorded in the information page of the drawer.

The information page of the drawer includes a drawer name, a drawer state icon, and a layout diagram of the drawer.

The drawer name is configured to represent the type of the drawer.

The drawer state icon is configured to represent the state of the drawer.

The layout diagram of the drawer includes the type of the sample carrier, a sample carrier icon, and a placement position icon. A display element of the sample carrier icon is configured to represent the state of the actual sample carrier. A display element of the placement position icon is configured to represent the state of the actual placement position. The layout of the sample carrier icon corresponds to the layout of the actual sample carrier in the drawer. The layout of the placement position corresponds to the layout of the actual placement position in the sample carrier.

The information page of the drawer further includes a display mode switching control. The method for processing information of the sample processing module further includes the following operations.

Displaying the information page of each drawer in a balanced manner is switched to highlighting the information page of the corresponding drawer in the first area in response to detecting an operation on the display mode switching control of the information page of any drawer in a balanced display state.
And/or,
high lighting the information page of the current drawer is switched to displaying the information page of each drawer in a balanced manner in the first area in response to detecting an operation on the display mode switching control of the information page of the drawer in a highlighted state.

In some embodiments, the drawer is configured to accommodate a sample carrier, and the sample carrier includes a plurality of placement positions for loading the sample tubes. The first area is configured to display the information page of the drawer, and the information of the drawer is recorded in the drawer of the information page.

The information page of the drawer includes a first information page and a second information page.

The method for processing information of the sample processing module further includes: highlighting the first information page of the first drawer in response to the first drawer being opened, and the first information page includes at least one of a type of the drawer, a state icon of the drawer, a type of each sample carrier in the drawer, and an unknown state icon of each sample carrier in the drawer. The unknown state icon of the sample carrier represents an unknown state of the sample carrier in the drawer.

The method for processing information of the sample processing module further includes: displaying the second information page of each drawer of the sample storage device in response to all the drawers being in the closed state, and the second information page includes a drawer name, a drawer state icon, and a layout diagram of the drawer.

The drawer name is configured to represent the type of the drawer.

The drawer state icon is configured to represent the state of the drawer.

The layout diagram of the drawer includes the type of the sample carrier, a sample carrier icon, and a placement position icon. A display element of the sample carrier icon is configured to represent the state of the actual sample carrier. A display element of the placement position icon is configured to represent the state of the actual placement position. The layout of the sample carrier icon corresponds to the layout of the actual sample carrier in the drawer. The layout of the placement position corresponds to the layout of the actual placement position in the sample carrier.

In some embodiments, the operation of highlighting the information of the drawer includes any of the following manners.

In the first manner, the information to be highlighted of the drawer is enlarged and displayed, and the information of the other drawers is partially or completely covered.

In the second manner, the information to be highlighted of the drawer is enlarged and displayed, and the information of the other drawers is stacked below the enlarged and displayed information of the drawer.

In some embodiments, the operation of displaying the information of each drawer of the sample storage device in a balanced manner includes the following operations.

The first area is divided into a corresponding number of sub-areas in equal proportion according to the number of the drawers of the sample storage device.

According a physical position of the drawer, each sub-area corresponds to the drawer, and the information page of the corresponding drawer in each sub-area is displayed.

In some embodiments, the display interface of the human-computer interaction device further includes a second area for displaying an operation control. The operation control includes at least one of an initialization control, a startup control, and a shutdown control.

In some embodiments, the method for processing information of the sample processing module further includes: controlling a mechanical arm to count the state of the placement position of the sample carrier of the sample output drawer in response to detecting that the opened sample output drawer is closed, and updating the information of the sample output drawer according to a counting result.

In some embodiments, the method for processing information of the sample processing module further includes: controlling the mechanical arm to count a target placement position of the sample carrier storing the sample tube in the sample output drawer, and detecting whether the sample tube still exists in the target placement position. The target placement position is any placement position in the sample carrier where the sample tube is placed.

The method for processing information of the sample processing module further includes: updating the state of each placement position of the sample carrier to no sample tube in a case where no sample tube is detected in the target placement position of the sample carrier storing the sample tube in the sample output drawer.

According the above method for processing information of the sample processing module, in response to the drawer being opened, the information of the drawer is highlighted in the first area of the human-computer interaction device, which can play a role in enlarging the information for prompting the opened drawer, so that a user can focus on the currently opened drawer. In response to the drawer being pushed to the closed position and all the drawers being in the closed state, the information of each drawer of the sample storage device is displayed in a balanced manner, so that the information of each drawer can be prompted without distinction.

It should be understood that, although the steps in the flowchart involved in the above embodiments are shown sequentially as indicated by arrows, but they are not necessarily performed sequentially in an order indicated by the arrows. Unless otherwise specified herein, there is no strict order restriction on the execution of these steps, and these steps can be executed in other orders. Moreover, at least some of the steps in the flowchart involved in the above embodiments may include a plurality of steps or stages, which are not necessarily executed at the same moment, but may be executed at different moments, and the sequence of execution of these steps or stages is not necessarily performed sequentially, but may be executed alternately or alternately with other steps or at least part of steps or stages in other steps.

The present disclosure further provides a computer device, including a memory and a processor. The memory stores a computer, and the processor executes the computer program to implement the steps of the above method for processing information of the sample processing module.

The present disclosure further provides a computer-readable storage medium, on which a computer program is stored. The computer program implements, when executed by a processor, the steps of the above method for processing information of the sample processing module.

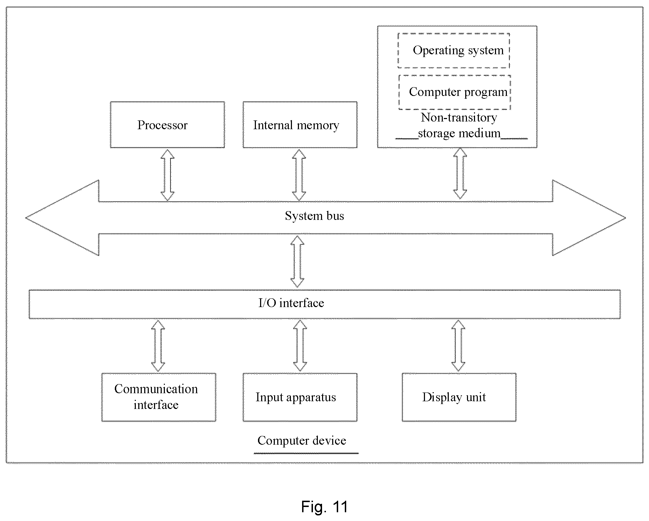

In some embodiments, a computer device is provided. The computer device may be the above processor, and an internal structural diagram thereof may be shown in Fig. 11. The computer device includes a processor, a memory, a communication interface, a display screen, and an input apparatus which are connected through a system bus. The processor of the computer device is configured to provide computing and control capabilities. The memory of the computer device includes a non-transitory storage medium and an internal memory. The non-transitory storage medium stores an operating system and a computer program. The internal memory provides an environment for the operation of an operating system and the computer program in the non-transitory storage medium. The communication interface of the computer device is configured to communicate with an external terminal in a wired or wireless manner, and the wireless manner may be implemented through WIFI, a mobile cellular network, Near Field Communication (NFC) or other technologies. The computer program is executed by the processor to implement an information processing method of a sample processing module. The display screen of the computer device may be a liquid crystal display screen or an electronic ink display screen, and the input apparatus of the computer device may be a touch layer covering the display screen, or a key, a trackball or a touchpad provided on a housing of the computer device, or an external keyboard, touchpad or mouse, etc.

Those skilled in the art may understand that the structure shown in Fig. 11 is merely a block diagram of a partial structure related to the solution of the present disclosure, and does not constitute a limitation on the computer device to which the solution of the present disclosure is applied. The specific computer device may include more or fewer components than shown in the figure, or combine certain components, or have a different arrangement of components.

Those of ordinary skill in the art may understand that implementation of all or part of the processes in the above embodiment method may be completed by instructing related hardware through a computer program, and the computer program may be stored in a non-transitory computer-readable storage medium. The computer program, when executed, may include the processes of the embodiments of the above methods. Any reference to a memory, a database, or another medium used in the embodiments provided in the present disclosure may include at least one of a non-transitory memory and a volatile memory. The non-transitory memory may include a Read-Only Memory (ROM), a magnetic tape, a floppy disk, a flash, an optical memory, a high-density embedded non-transitory memory, a Resistive Random Access Memory (ReRAM), a Magnetoresistive Random Access Memory (MRAM), a Ferroelectric Random Access Memory (FRAM), a Phase Change Memory (PCM), a graphene memory, etc. The volatile memory may include a Random Access Memory (RAM) or an external high-speed cache etc. As illustrative rather than restrictive, RAMs may be in various forms, such as a Static RAM (SRAM), or a Dynamic RAM (DRAM), etc. The database involved in various embodiments provided in the present disclosure may include at least one of a relational database and a non-relational database. The non-relational database may include, but is not limited to, a distributed database based on a blockchain, etc. The processor involved in various embodiments provided in the present disclosure may be a general-purpose processor, a central processing unit, a graphics processor, a digital signal processor, a programmable logic unit, a data processing logic unit based on quantum computing, etc., but are not limited thereto.

The technical features of the above-described embodiments may be arbitrarily combined. For the sake of brevity of description, all possible combinations of the technical features in the above embodiments are not described. However, as long as there is no contradiction between the combinations of these technical features, all should be considered as the scope of this description.

The above embodiments are merely illustrative of several implementations of the present disclosure with specific and detailed description, and are not to be construed as limiting the patent scope of the present disclosure. It is to be noted that a number of variations and modifications may be made by those of ordinary skill in the art without departing from the conception of the present disclosure, and all fall within the scope of protection of the present disclosure. Therefore, the scope of protection of the present disclosure should be determined by the appended claims.

## Claims

1. A sample processing module, comprising:
a sample storage device, the sample storage device comprising a plurality of drawers for storing sample tubes;
a state detection device of each drawer, configured to detect a state of drawer corresponding to the state detection device;
a human-computer interaction device, wherein a display interface of the human-computer interaction device at least comprises a first area, configured to display information of the drawer; and
a processor, configured to acquire the state of the drawer, and switch from currently displayed information of the drawer to highlighting a information of a first drawer in the first area in response to detecting that the first drawer is opened, wherein the first drawer is any drawer of the sample storage device.

2. The sample processing module as claimed in claim 1, wherein the processor is further configured to switch, in a case where all the drawers of the sample storage device are in a closed state, from displaying the information of each drawer of the sample storage device in a balanced manner to highlighting the information of the first drawer in the first area in response to detecting that the first drawer is opened.

3. The sample processing module as claimed in claim 1, wherein the processor is further configured to switch, in a case where a second drawer is already open in the sample storage device, from highlighting the information of the second drawer to highlighting the information of the first drawer in the first area in response to detecting that the first drawer is opened.

4. The sample processing module as claimed in claim 3, wherein the processor is further configured to:
switch, in a case where the number of the second drawers that are already open is one, from highlighting the information of the first drawer to highlighting the information of the second drawer in the first area in response to detecting that the first drawer is closed;
or,
switch, in a case where the number of the second drawers that are already open is at least two, from highlighting the information of the first drawer to highlighting the information of a target drawer in the first area in response to detecting that the first drawer is closed, wherein the target drawer is either the drawer whose opening time is closest to the current time among the at least two second drawers or the drawer with the highest priority among the at least two second drawers.

5. The sample processing module as claimed in claim 1, wherein the processor is further configured to switch from highlighting the information of the first drawer to displaying the information of each drawer of the sample storage device in a balanced manner in the first area in response to detecting that the first drawer is closed and all the drawers of the sample storage device are in a closed state after the first drawer is closed.

6. The sample processing module as claimed in any one of claims 1 to 5, wherein the drawer is configured to accommodate a sample carrier, and the sample carrier comprises a plurality of placement positions for loading sample tubes; and the information of the drawer comprises at least one of a type of the drawer, a state of the drawer, a type of the sample carrier in the drawer, a state of the sample carrier in the drawer, a layout of the sample carrier in the drawer, a state of each placement position in the sample carrier, and a layout of the placement position in the sample carrier.

7. The sample processing module as claimed in claim 6, wherein the first area is configured to display an information page of the drawer, and the information of the drawer is recorded in the information page of the drawer;
the information page of the drawer comprises a drawer name, a drawer state icon, and a layout diagram of the drawer;
the drawer name is configured to represent the type of the drawer;
the drawer state icon is configured to represent the state of the drawer;
the layout diagram of the drawer comprises the type of the sample carrier, a sample carrier icon, and a placement position icon; a display element of the sample carrier icon is configured to represent the state of the actual sample carrier; a display element of the placement position icon is configured to represent the state of the actual placement position; the layout of the sample carrier icon corresponds to the layout of the actual sample carrier in the drawer; and the layout of the placement position corresponds to the layout of the actual placement position in the sample carrier.

8. The sample processing module as claimed in claim 7, wherein the information page of the drawer further comprises a display mode switching control; and the processor is further configured to:
switch from displaying the information page of each drawer in a balanced manner to highlighting the information page of corresponding drawer in the first area in response to detecting an operation on the display mode switching control of the information page of any drawer in a balanced display state;
and/or,
switch from highlighting the information page of the current drawer to displaying the information page of each drawer in a balanced manner in the first area in response to detecting an operation on the display mode switching control of the information page of the drawer in a highlighted state.

9. The sample processing module as claimed in any one of claims 1 to 5, wherein the drawer is configured to accommodate a sample carrier, and the sample carrier comprises a plurality of placement positions for loading the sample tubes; the first area is configured to display an information page of the drawer, and the information of the drawer is recorded in the information page of the drawer;
the information page of the drawer comprises a first information page and a second information page;
the processor is further configured to highlight the first information page of the first drawer in response to the first drawer being opened, and the first information page comprises at least one of a type of the drawer, a state icon of the drawer, a type of each sample carrier in the drawer, and an unknown state icon of each sample carrier in the drawer; the unknown state icon of the sample carrier represents an unknown state of the sample carrier in the drawer;
the processor is further configured to display the second information page of each drawer of the sample storage device in response to all the drawers being in the closed state, and the second information page comprises a drawer name, a drawer state icon, and a layout diagram of the drawer;
the drawer name is configured to represent the type of the drawer;
the drawer state icon is configured to represent the state of the drawer;
the layout diagram of the drawer comprises the type of the sample carrier, a sample carrier icon, and a placement position icon; a display element of the sample carrier icon is configured to represent the state of the actual sample carrier; a display element of the placement position icon is configured to represent the state of the actual placement position; the layout of the sample carrier icon corresponds to the layout of the actual sample carrier in the drawer; and the layout of the placement position corresponds to the layout of the actual placement position in the sample carrier.

10. The sample processing module as claimed in any one of claims 1 to 5, wherein the highlighting the information of the drawer comprises any one of the following manners:
the first manner: enlarging and displaying the information to be highlighted of the drawer, and partially or completely covering the information of the other drawers; and
the second manner: enlarging and displaying the information to be highlighted of the drawer, and stacking the information of the other drawers below the enlarged and displayed information of the drawer.

11. The sample processing module as claimed in claim 2 or 5, wherein the displaying the information of each drawer of the sample storage device in a balanced manner comprises:
dividing the first area into a corresponding number of sub-areas in equal proportion according to the number of the drawers of the sample storage device; and
according to a physical position of the drawer, enabling each sub-area to correspond to the drawer, and displaying the information page of corresponding drawer in each sub-area.

12. The sample processing module as claimed in claim 1, wherein the display interface of the human-computer interaction device further comprises a second area for displaying an operation control; and the operation control comprises at least one of an initialization control, a startup control, and a shutdown control.

13. The sample processing module as claimed in claim 6, further comprising a mechanical arm; the type of the drawer comprises a sample output drawer; and
the processor is further configured to control the mechanical arm to count the state of the placement position of the sample carrier of the sample output drawer in response to detecting that the opened sample output drawer is closed, and update the information of the sample output drawer according to a counting result.

14. The sample processing module as claimed in claim 13, wherein the processor is further configured to control the mechanical arm to count a target placement position of the sample carrier storing the sample tube in the sample output drawer, and detect whether the sample tube still exists in the target placement position; and the target placement position is any placement position in the sample carrier where the sample tube is placed; and the processor is further configured to update the state of each placement position of the sample carrier to no sample tube in response to not detecting the sample tube in the target placement position.

15. A method for processing information of a sample processing module, comprising:
detecting a state of each drawer of the sample processing module; and
switching from currently displayed information of the drawer to highlighting a information of a first drawer in a first area of a human-computer interaction device in response to detecting that the first drawer is opened.
